# EUROPEAN PATENT SPECIFICATION

(11) **EP 3 344 603 B1**
(45) Date of publication and mention of the grant of the patent: **30.09.2020**
(21) Application number: 16762955.9
(22) Date of filing: 18.08.2016
(51) Int. Cl.: C07D 211/58

(54) **A PRODUCTION METHOD OF 1-(4-FLUOROBENZYL)-3-(4-ISOBUTOXYBENZYL)-1-(1-METHYLPIPERIDIN- 4 YL)UREA AND ITS DEUTERATED ANALOGS NOT CONTAINING DIMERIC IMPURITIES**
HERSTELLUNGSVERFAHREN FÜR 1-(4-FLUOROBENZYL)-3-(4-ISOBUTOXYBENZYL)-1-(1-METHYLPIPERIDIN-4-YL)HARNSTOFF UND DESSEN DEUTERIERTER ANALOGA OHNE DIMERE VERUNREINIGUNGEN
PROCÉDÉ DE PRODUCTION DE 1-(4-FLUOROBENZYL)-3-(4-ISOBUTOXYBENZYL)-1-(1-MÉTHYLPIPÉRIDIN-4-YL)URÉE ET DE SES ANALOGUES DEUTÉRÉS NE CONTENANT PAS D'IMPURETÉS SOUS FORME DE DIMÈRES

(30) Priority: 02.09.2015 CZ 20150601
(43) Date of publication of application: 11.07.2018
(73) Proprietor: Zentiva, K.S., 102 37 Praha 10 (CZ)
(72) Inventor: RADL, Stanislav, 250 84 Kvetnice (CZ); STACH, Jan, 104 00 Praha (CZ); KLECAN, Ondrej, 282 01 Cesky Brod (CZ)
(74) Representative: Ellis, Robin Patrick
(86) International application number: PCT/CZ2016/000093
(87) International publication number: WO 2017/036432

(56) References cited:
- WO-A1-2008/141057
- WO-A1-2014/085362
- FR-A1- 2 921 926

## Description

### Field of the Invention

The invention relates to an improved production method of 1-(4-fluorobenzyl)-3-(4--isobutoxybenzyl)-1-(1--methylpiperidin-4-yl)urea of formula **I**, known under the generic name of pimavanserin.

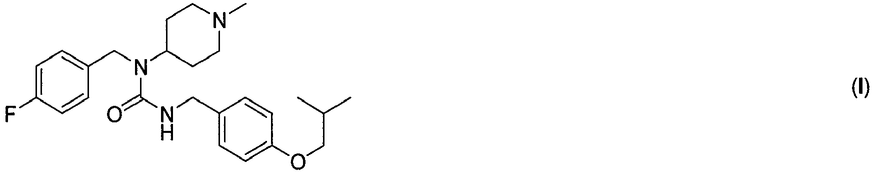

Pimavanserin is an inverse agonist of the serotonin 5-HT_{2A} receptor and is currently in the tartrate form in stage III of clinical trial for the treatment of psychosis in patients suffering from Parkinson's disease and has potential to become part of the therapy of other psychotic conditions.

### Background Art

Pimavanserin of formula **I** is produced, according to the published patent applications (WO2004064738, WO2006036874, WO2006037043), from the input *N*-(4-fluorobenzyl)-1-methylpiperidine-4-amine of formula **II** through a reaction with 1-isobutoxybenzyl isocyanate of formula **III**, providing crude pimavanserin (Diagram 1).

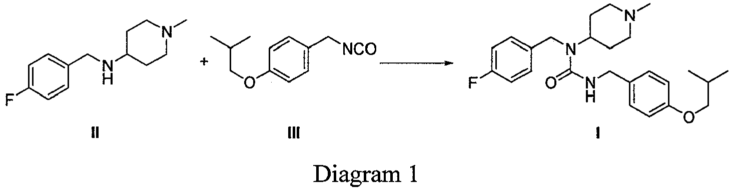

The above mentioned patents also describe synthesis of *N*-(4-fluorobenzyl)-1-methylpiperidine-4-amine of formula **II** by reductive alkylation of 4-fluorobenzylamine of formula **IV** with 1-methylpiperidin-4-one of formula **V**. According to the above mentioned patents, the other component for synthesis of pimavanserin of formula **I**, 4-isobutoxybenzyl isocyanate of formula **III** can be obtained through a reaction of 4-isobutoxybenzylamine of formula **VII**, which can be prepared in two stages from 4-isobutoxybenzaldehyde of formula **VI** with phosgene or its equivalents. Another described synthesis of 4-isobutoxybenzyl isocyanate of formula **III** is based on 2-(4-isobutoxyphenyl)acetic acid of formula **VIII**, which provides, through a reaction with diphenyl phosphoryl azide (DPPA), the isocyanate of formula **III** (Diagram 2).

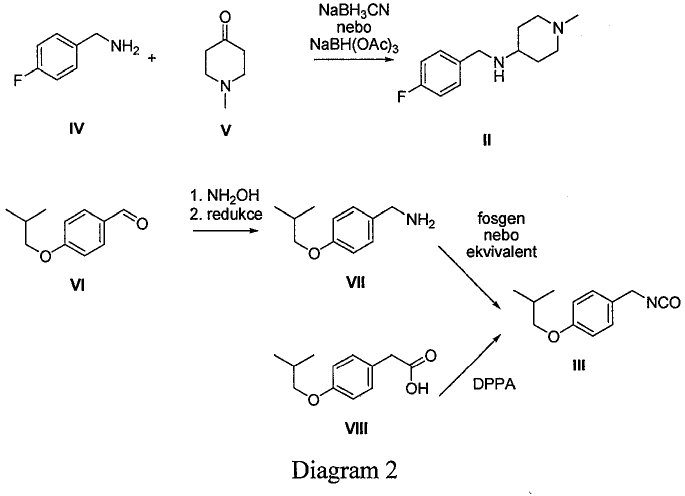

However, the above mentioned procedures of pimavanserin synthesis feature a number of disadvantages. The main disadvantage comprises the use of 4-isobutoxybenzyl isocyanate of formula **III**, which emits, as all the substances of this type, unpleasant smell and is highly reactive. Another disadvantage is that the described procedures of its synthesis either use the highly toxic phosgene, or the also toxic diphenyl phosphoryl azide (DPPA), which is additionally as all azides potentially explosive.

Another disadvantage of the said procedure is the fact that crude pimavanserin of formula **I** contains some impurities the removal of which leads to considerable losses. Namely, as unpleasant impurities from this point of view *N*-(4-fluorobenzyl)-1-methylpiperidine-4-amine of formula **II** and 1,3-bis(4-isobutoxybenzyl)urea of formula **IX** are mentioned.

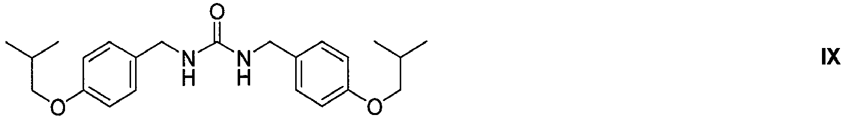

Partly deuterated analogs of pimavanserin are also described that exhibit, based on the kinetic isotope effect, lower sensitivity to metabolic degradation and can thus have the corresponding therapeutic advantages as a lower dosage, higher effect, lower inter-individual deviations and more (WO2008141057). However, their synthesis is analogous to the above mentioned procedures described in the published patent applications WO2004064738, WO 2006036874 and WO2006037043 with the use of partly deuterated input substances.

### Disclosure of the Invention

The invention relates to a new efficient preparation method of highly pure 1-(4-fluorobenzyl)-3-(4-isobutoxybenzyl)-1-(1-methylpiperidin-4-yl)urea - pimavanserin that is based on the known intermediates of its synthesis, namely *N*-(4-fluorobenzyl)-1-methylpiperidine-4-amine of formula **II** and 4-isobutoxybenzaldehyde of formula **VI**. The method consists, in the first stage, in conversion of *N*-(4-fluorobenzyl)-1-methylpiperidine-4-amine of formula **II** to 1-(4-fluorobenzyl)-1-(1-methylpiperidin-4-yl)urea of formula **X** through a reaction with isocyanic acid. It can be released *in situ* from commercially available safe salts, e.g.alkaline isocyanates, preferably KNCO or NaNCO.

Reductive alkylation of 1-(4-fluorobenzyl)-1-(1-methylpiperidin-4-yl)urea of formula **X** with 4-isobutoxybenzaldehyde of formula **VI** under the conditions described in this invention can then be used to obtain crude pimavanserin that contains a very low quantity of the impurity *N-*(4-fluorobenzyl)-1-methylpiperidine-4-amine of formula **II** and with regard to the method of synthesis does not contain the disubstituted impurity of formula **IX** (Diagram 3).

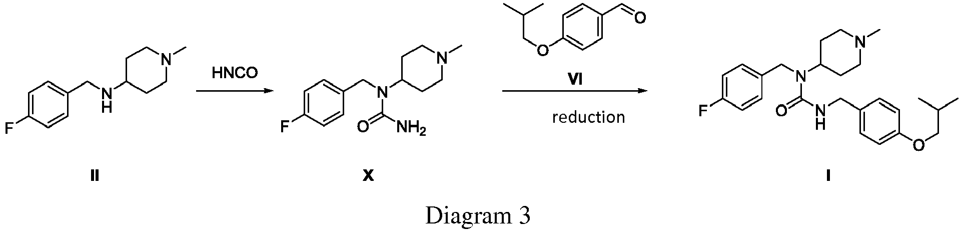

The invention relates to a new efficient preparation method of highly pure 1-(4-fluorobenzyl)-3-(4-isobutoxybenzyl)-1-(1-methylpiperidin-4-yl)urea - pimavanserin of formula **I**, which does not contain the amine of formula **II**, or the doubled impurity of formula **IX**.

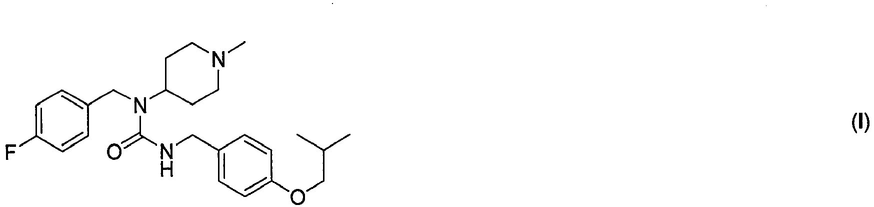

The method is based on the known *N*-(4-fluorobenzyl)-1-methylpiperidine-4-amine of formula **II**, which provides, through a reaction with cyanic acid, high yields of 1-(4-fluorobenzyl)-1-(1-methylpiperidin-4-yl)urea of formula **X**. The reactions can be efficiently conducted in a number of organic solvents, in their mixtures with water, or just in aqueous solutions. Especially suitable solvents for this purpose are aromatic hydrocarbons (preferably toluene), C1-C5 alkyl alcohols, C1-C5 alkyl carboxylic acids, aprotic solvents as dialkyl amides of carboxylic acids (preferably dimethyl formamide, dimethyl acetamide, *N*-methyl pyrrolidone, nitriles of carboxylic acids (preferably acetonitrile), or sulfoxides (preferably dimethyl sulfoxide). It is especially advantageous to use a mixture of one or more of the said solvents with water wherein the water content can be in the whole concentration range, i.e. from 0 to 100%. In some embodiments it is especially suitable to carry out the reaction in an aqueous environment. Isocyanic acid can be released *in situ* from alkaline cyanates, preferably KCNO or NaCNO with a number of organic or inorganic acids. The input cyanate can be advantageously used in a surplus, an advantageous surplus amounting to 1.1 to 3 equivalents, the most advantageous one being in the range of 1.25 to 2 equivalents while the cyanate can be added to the reaction mixture all at once or gradually in several doses. As the organic acids carboxylic acids can be used (preferably C2-C8 alkyl acids, aromatic acids), or sulfonic acids (preferably C2-C8 alkyl sulfonic acids, aromatic sulfonic acids). As the inorganic compounds virtually all common inorganic acids can be used, preferably hydrochloric acid or sulfuric acid. The reaction can also be carried out without the use of acids; in this case it is advantageous to use the input *N*-(4-fluorobenzyl)-1--methylpiperidine-4--amine of formula **II** in the form of a salt with the above mentioned acids. Using the dihydrochloride of the amine of formula **II** is especially convenient. Depending on the used solvents, the reaction can be carried out in a wide range of temperatures from the melting point to the boiling point, preferably in the range from 0°C to 75°C, more preferably in the range from 20 to 50°C, most preferably at the laboratory temperature.

The term "C1-C5 alkyl", as it is used here, means a monodentate hydrocarbon residue containing one to five carbon atoms in a chain that can be linear, branched or cyclic. Examples of the alkyl groups comprise, but are not limited to methyl, ethyl, *n*-propyl, isopropyl, *n*-butyl, isobutyl, tert-butyl and cyclopentyl. The term "C2-C8 alkyl", as it is used here, means a monodentate hydrocarbon residue containing two to eight carbon atoms in a chain that can be linear, branched or cyclic. Examples of the alkyl groups comprise, but are not limited to ethyl, *n*-propyl, isopropyl, *n*-butyl, isobutyl, tert-butyl, *n*-pentyl, cyclopentyl and cyclohexyl.

Reductive alkylation of amines (reductive amination of aldehydes and ketones) is a common reaction used for synthesis of a number of secondary or tertiary amines, including the amine of formula **II**. Common reduction agents used for this reaction are NaBH₃CN, NaBH(OAc)₃, or under certain conditions also NaBH₄. On the other hand, reference literature describes several unsuccessful attempts at reductive alkylation of unsubstituted urea or its mono- and dialkyl derivatives (Abdel-Magid et al.: J. Org. Chem. 1996, 61, 3849). It is only recently that the first successful reductive alkylations of urea derivatives were described (Armstrong et al.: Tetrahedron Lett. 1998, 38, 1531; Xu et al.: Tetrahedron Lett. 1998, 39, 1107; Dubé, Scholte: Tetrahedron Lett. 1999, 40, 2295; Patel et al.: Tetrahedron Lett. 2009, 50, 5975; El Dine et al.: Eur. J. Org. Chem. 2013, 5445). At the same time, the first described successful reaction nearly exclusively concerned alkylation of urea or monosubstituted urea.

According to the invention, reductive amination can be carried out through a reaction of 1-(4-fluorobenzyl)-1-(1--methylpiperidin-4-yl)urea of formula **X** with 4-isobutoxybenzaldehyde of **formula VI**, or under conditions when this aldehyde is released (acidic environment) also with its equivalents as the respective bisulphidic adduct of formula **XI** or the respective acetals of formula **XII**, 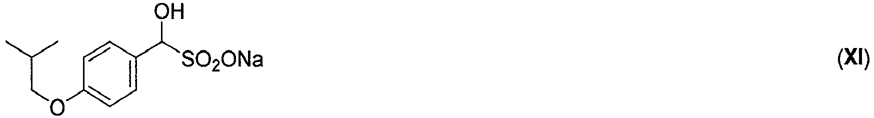 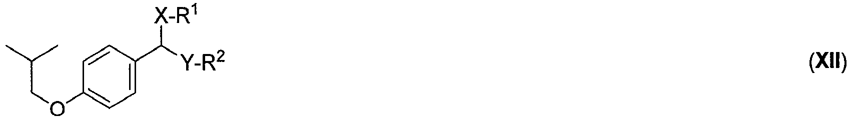 where X and Y can be independently O or S and where R¹ and R² can be a C1-C6 alkyl, or where R¹,R² can form a 5- to 6-membered cycle together with the X-C-Y structure they are connected to.

The entire reductive alkylation can be conducted with the use of NaBH₄ as a reduction agent under conditions when the respective imine is formed. What is especially advantageous is the use of activation agents acting as catalysts in the production of imines on the one hand and on the other hand as catchers of generated water of the acyl halide type, preferably acetylchloride, acyl anhydrides, preferably acetic anhydride, sulfonyl halides, preferably methane sulfonyl chloride, sulfone anhydrides, preferably methane sulfonyl anhydride, or trialkyl silyl halides, preferably trimethyl silyl chloride. Another option consists in using a Lewis acid as the activation agent, preferably selected from titanium alkoxides, preferably Ti(Oi-Pr)₄. Besides NaBH₄, the above mentioned reduction agents NaBH₃CN and NaBH(OAc)₃ can also be used with these activators.

What is especially advantageous for the reduction alkylation in accordance with the invention is to use reductive agents containing Si-H as the reductive group, preferably trialkylsilanes as triethylsilane, tetraalkyldisiloxanes, preferably tetramethyldisiloxane (TMDS), pentaalkyldisiloxanes, preferably pentamethyldisiloxane, hexaalkyltrisiloxanes, preferably 1,1,3,3,5,5-hexamethyltrisiloxane, cyclic hydrosiloxanes, preferably 2,4,6,8-tetramethylcyclotetrasiloxane or 2,4,6,8,10-pentamethylcyclopentasiloxane, or polymeric siloxanes containing the Si-H group, preferably polymethylhydrosiloxane (PMHS). For sufficient reactivity these agents are activated either by Lewis acids, preferably Ti(Oi-Pr)₄, BF₃, BCl₃, FeCl₃, InCl₃, BiCl₃, or Brönsted acids, preferably CF₃COOH or CF₃SO₂OH.

As the solvents for the synthesis of pimavanserin of formula **I** by reductive alkylation in accordance with the present invention a number of solvents can be used, including C1-C5 alkyl aliphatic acids (preferably acetic acid), haloalkanes (preferably dichloromethane), hydrocarbons (preferably toluene), ethers (preferably dimethoxyethane, tetrahydrofuran or 2-methyltetrahydrofuran), nitriles (preferably acetonitrile), or mixtures of the above mentioned solvents. The term "C1-C5 alkyl", as it is used here, means a monodentate hydrocarbon residue containing one to five carbon atoms in a chain that can be linear, branched or cyclic. Examples of the alkyl groups comprise, but are not limited to methyl, ethyl, *n*-propyl, isopropyl, *n*-butyl, isobutyl, tert-butyl and cyclopentyl.

The above described method of synthesis of pimavanserin of formula **I** is also applicable to the preparation of the respective partly deuterated analogs. Using the methods for non-deuterated substances described in the published patent applications WO2004064738, WO2006036874, WO2006037043 partly deuterated intermediates **II-D1, II-D3, II-D4, II-D4', II-D7, II-D8** and **VI-D9** have been prepared from commercially available deuterated ingredients.

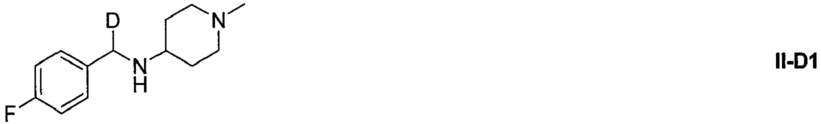

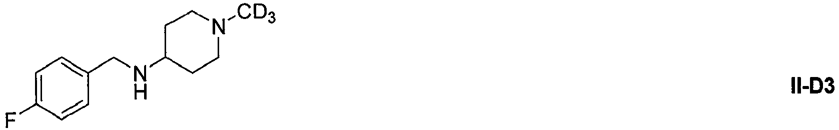

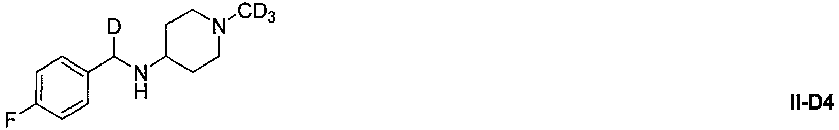

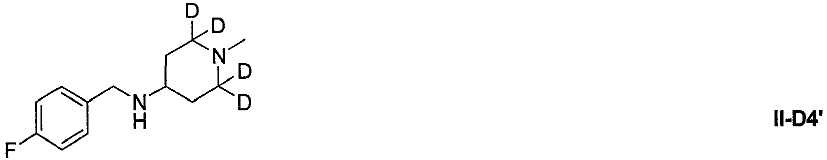

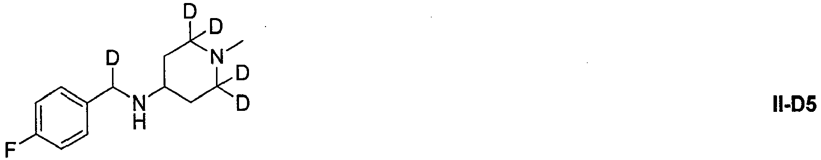

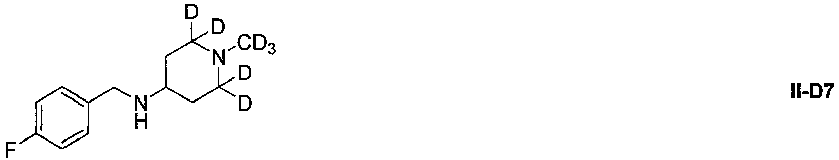

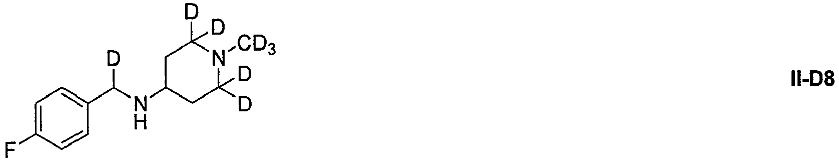

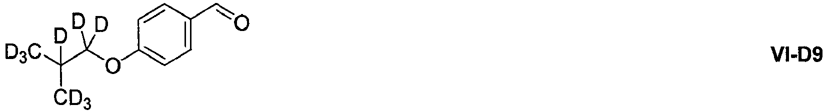

Using a mutual reaction of the intermediates **II-D** with the intermediate of formula **VI** with the use of methods according to the present invention partly deuterated analogs of pimavanserin of formulae **I-D1, I-D3, I-D4, I-D4', I-D7** and **I-D8** have been prepared.

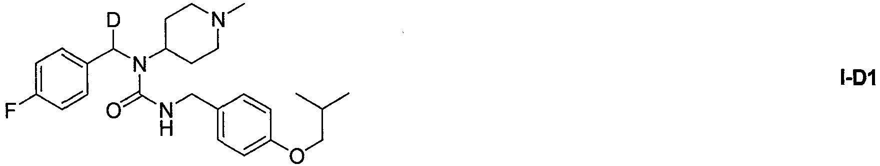

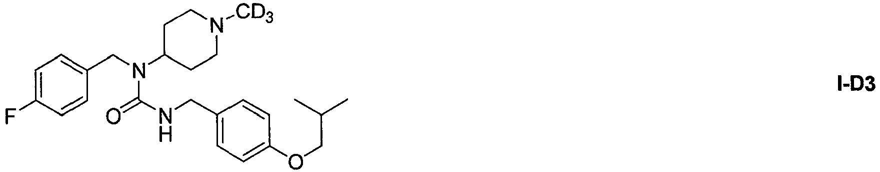

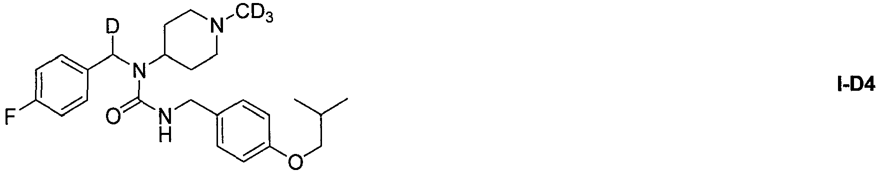

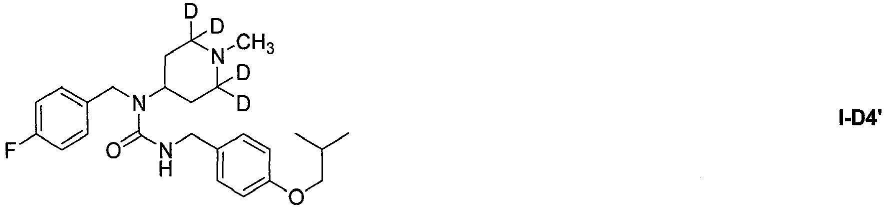

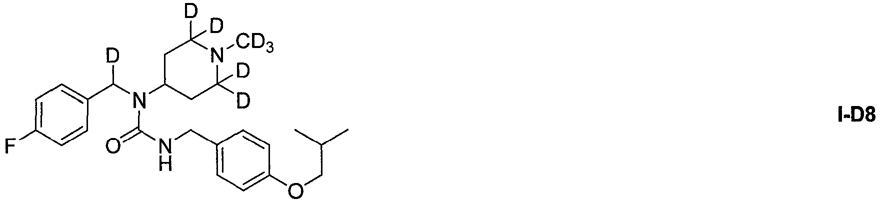

A mutual reaction of the intermediates of formulae **II** and **II-D** with the intermediate of formula **VI-D8** can be used to prepare, with the use of methods according to the present invention, further deuterated analogs of pimavanserin, which are represented by the formulae **I-D16 and I-D17**.

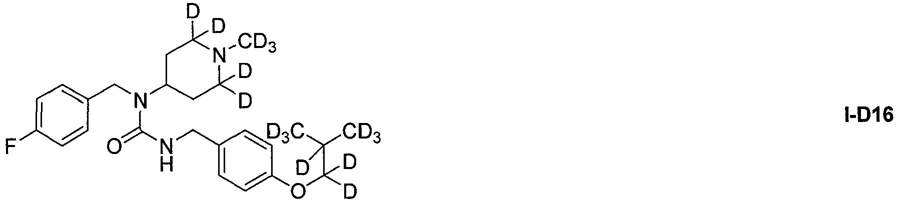

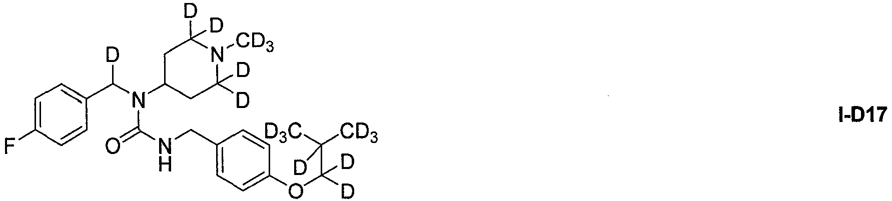

With the use of sodium borodeuteride in reductive alkylation of the substances of formula **II** or **II-D** with **VII** and **VI-D9** similar partly deuterated derivatives represented by the substances of formulae **I-D17'** and **I-D18** can be obtained.

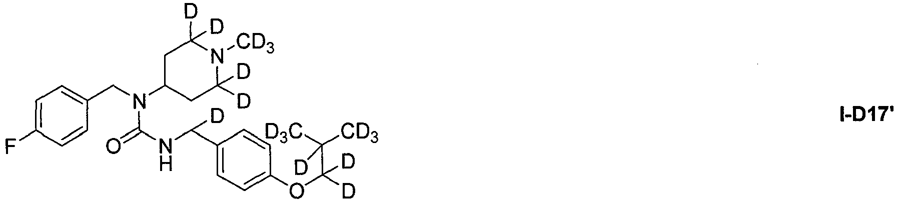

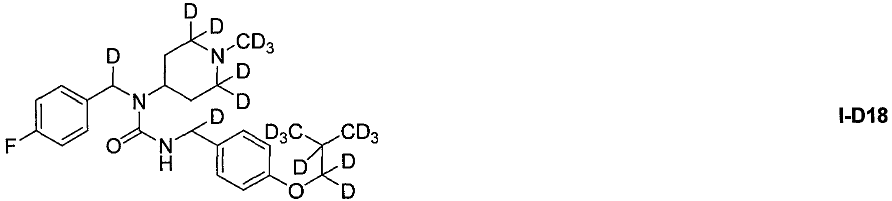

This account does not limit the possibilities of the method in accordance with the present invention to synthesize further deuterated analogs of pimavanserin in any respect, they only illustrate usefulness of the method.

The invention is clarified in a more detailed way using the embodiment examples below. These examples illustrate the improvement of the method in accordance with the invention, having exclusively an illustrative character, and do not restrict the scope of the invention in any respect.

### Examples

### Example 1

### 1-(4-Fluorobenzyl)-1-(1-methylpiperidin-4-yl)urea of formula X

To a solution of *N*-(4-fluorobenzyl)-1-methylpiperidine-4-amine (**II;** 2.22 g, 10 mmol) in acetic acid (20 ml) NaCNO (1.63 g, 25 mmol) was added and the mixture was stirred at the laboratory temperature for 24 h. Then, the reaction mixture was poured into 2 M NaOH (200 ml) and extracted with dichloromethane (5 x 50 ml). The united extracts were washed with salt brine (2 x 50 ml) and dried with Na₂SO₄ and after evaporation the amount of 2.4 g of a colorless evaporation product was obtained whose trituration with Et₂O provided 2.1 g (79%) of a white solid substance with the melting point of 114-117°C. ¹H NMR (CDCl₃, 500.13 MHz, 298 K): 7.22 (dd, 2H, *J* = 8.7, 5.2 Hz, Ar*H*), 7.02 (t, 2H, *J* = 8.7 Hz, Ar*H*), 4.47 (brs, 2H, N*H₂*), 4.37 (s, 2H, Ar-C*H*₂-N), 4.25 - 4.20 (m, 1H, N-C*H*), 2.89 - 2.84 (m, 2H, (C*H₂*)₂-N-CH₃), 2.25 (s, 3H, N-C*H₃*), 2.06 (td, 2H, *J* = 11.8, 2.7 Hz, (C*H₂*)₂-N-CH₃), 1.74 - 1.62 (m, 4H, CH-(C*H₂*)₂). ¹³C NMR (CDCl₃, 125.76 MHz, 298 K): 161.99 (*C*-F), 159.01 (*C*=O), 133.71 (quart Ar*C*), 127.61 (Ar*C*H), 115.78 (ArCH), 55.12 ((*C*H₂)₂-N-CH₃), 52.32 N-*C*H), 46.00 (N-*C*H₃), 45.56 (Ar-*C*H₂-N), 29.92 (CH-(*C*H₂)₂).

### Example 2

### 1-(4-Fluorobenzyl)-1-(1-methylpiperidin-4-yl)urea of formula X

To a solution of *N*-(4-fluorobenzyl)-1-methylpiperidine-4-amine (**II;** 2.22 g, 10 mmol) in methanol (10 ml) 1M solution of H₂SO₄ (10 ml) was added, the mixture was cooled down to 10°C and then a solution of NaCNO (1.63 g, 25 mmol) in 10 ml of water was added. The mixture was stirred for 2 h at the laboratory temperature and then for 4 h at the temperature of 50°C. After evaporation of methanol the mixture was alkalinized with 2 M NaOH to pH 8.0-8.5 and then extracted with dichloromethane (5 x 20 ml), the extract was washed with salt brine (10 ml) and dried with Na₂SO₄. The amount of 2.3 g of the evaporation product was obtained whose trituration with Et₂O provided 1.95 g (73.5%) of a white solid substance with the melting point of 115 to 117°C.

### Example 3

### 1-(4-Fluorobenzyl)-1-(1-methylpiperidin-4-yl)urea of formula X

To a mixture of *N*-(4-fluorobenzyl)-1-methylpiperidine-4-amine dihydrochloride (**II.2HCl;** 1.0 g, 3.4 mmol) in a mixture of water (5 ml) and isopropanol (15 ml) a solution of KCNO (0.55 g, 68 mmol) in 15 ml of water was added. The mixture was stirred for 1 h at the laboratory temperature and then for 8 h at the temperature of 70°C. After evaporation to a dry state the evaporation product was triturated with acetone, the separated KCl was aspirated and washed with acetone. After evaporation, the evaporation product (0.75 g) was triturated with pentane. The amount of 0.7 g (77.5%) of the product was obtained.

### Example 4

### 1-(4-Fluorobenzyl)-1-(1-methylpiperidin-4-yl)urea of formula X

To a solution of *N*-(4-fluorobenzyl)-1-methylpiperidine-4-amine (**II;** 2.22 g, 10 mmol) in toluene (30 ml) NaCNO (1.00 g, 15 mmol) was added and to the resulting stirred suspension CF₃COOH (1.7 g, 15 mmol) was added by dripping during 30 minutes and the mixture was stirred for 4 h. Then, another fraction of NaCNO (0.4 g, 6 mmol) and CF₃COOH (0.7 g, 6 mmol) was added and the mixture was stirred overnight (15 h). The reaction mixture was gradually washed with water (10 ml), 1 M NaOH (2x 5 ml) and salt brine (10 ml) and the solution was dried with Na₂SO₄. Trituration with pentane provided 1.9 g (72%) of the product.

### Example 5

### 1-(4-Fluorobenzyl)-3-(4-isobutoxybenzyl)-1-(1-methylpiperidin-4-yl)urea of formula X

To a solution of *N*-(4-fluorobenzyl)-1-methylpiperidine-4-amine dihydrochloride (**II.2HCl;** 7.4 g, 25 mmol) in water (75 ml) a solution of KCNO (3.2 g, 40 mmol) in 75 ml of water was added. The mixture was stirred for 24 h at the laboratory temperature (pH = 7.58). Then, more KCNO (0.8 g, 10 mmol) was added and the mixture was stirred for another 15 h (pH = 7.9). After the adjustment of pH with 1 M NaOH to pH = 9 solid NaCl (15 g) was added to the reaction mixture and after complete dissolution the reaction mixture was extracted with dichloromethane (10 x 50 ml). The extract was washed with salt brine and dried with MgSO₄. The vitreous evaporation product was triturated with Et₂O (50 ml) and after aspiration and washing with Et₂O the amount of 6.2 g (93.5%) of a white solid substance with the melting point of 115 to 117°C was obtained.

### Example 6

### 1-(4-Fluorobenzyl)-3-(4-isobutoxybenzyl)-1-(1-methylpiperidin-4-yl)urea of formula X

To a solution of *N-*(4-fluorobenzyl)-1-methylpiperidine-4-amine dihydrochloride (**II.2HCl;** 7.4 g, 25 mmol) in water (50 ml) a solution of KCNO (4 g, 50 mmol) in 50 ml of water was added. The mixture was stirred for 48 h at the laboratory temperature (pH = 8.38). Then, solid NaCl (30 g) was added to the reaction mixture and after complete dissolution the reaction mixture was extracted with dichloromethane (15 x 50 ml). The extract was washed with salt brine and dried with MgSO₄. The vitreous evaporation product was triturated with Et₂O (50 ml) and after aspiration and washing with Et₂O the amount of 5.6 g (84%) of a white solid substance with the melting point of 115 to 117°C was obtained.

### Example 7

### 1-(4-Fluorobenzyl)-3-(4-isobutoxybenzyl)-1-(1-methylpiperidin-4-yl)urea of formula X

To a suspension of *N*-(4-fluorobenzyl)-1-methylpiperidine-4-amine dihydrochloride (**II.2HCl;** 7.4 g, 25 mmol) in methanol (50 ml) KCNO (4 g, 50 mmol) was added and the mixture was stirred for 4 h at the laboratory temperature. The insoluble fraction was aspirated, washed with methanol (10 ml) and the filtrate was aspirated until dry. The obtained vitreous evaporation product was triturated with Et₂O (50 ml) and after aspiration and washing with Et₂O the amount of 6.0 g (90%) of a white solid substance with the melting point of 115 to 117°C was obtained.

### Example 8

### 1-(4-fluorophenyl)methyl-d1)-1-(1-methylpiperidin-4-yl)urea of formula X-D1

Using the procedure described in Example 3 with the use of the deuterated amine **II-D1** the product with the melting point of 114-116°C was obtained in the yield of 69%. ¹H NMR (CDCl₃, 500.13 MHz, 298 K): 7.20 (dd, 2H, *J* = 8.7, 5.2 Hz, Ar*H*), 7.05 (t, 2H, *J* = 8.7 Hz, Ar*H*), 4.30 (brs, 2H, N*H₂*), 4.32 (s, 1H, Ar-CD*H*-N), 4.27 - 4.18 (m, 1H, N-C*H*), 2.91 - 2.85 (m, 2H, (C*H₂*)₂-N-CH₃), 2.25 (s, 3H, N-C*H₃*), 2.08 (td, 2H, *J* = 11.8, 2.7 Hz, (C*H₂*)₂-N-CH₃), 1.76 - 1.60 (m, 4H, CH-(C*H₂*)₂).

### Example 9

### 1-(4-Fluorobenzyl)-1-(1-methyl-d3-piperidin-4-yl)urea of formula X-D3

Using the procedure described in Example 3 with the use of the deuterated amine **II-D3** the product with the melting point of 115-117°C was obtained in the yield of 73%. ¹H NMR (CDCl₃, 500.13 MHz, 298 K): 7.20 (dd, 2H, *J* = 8.7, 5.2 Hz, Ar*H*), 7.04 (t, 2H, *J* = 8.7 Hz, Ar*H*), 4.38 (brs, 2H, N*H₂*), 4.35 (s, 2H, Ar-C*H₂*-N), 4.25 - 4.15 (m, 1H, N-C*H*), 2.90 - 2.80 (m, 2H, (C*H₂*)₂-N-CH₃), 2.05 (td, 2H, *J* = 11.8, 2.7 Hz, (C*H₂*)₂-N-CH₃), 1.75 - 1.60 (m, 4H, CH-(C*H₂*)₂).

### Example 10

### 1-(4-Fluorobenzyl)-1-(1-methylpiperidin-2,2,6,4-d4-4-yl)urea of formula X-D4'

Using the procedure described in Example 3 with the use of the deuterated amine **II-D4'** the product with the melting point of 114-117°C was obtained in the yield of 77%.

### Example 11

### 1-(4-Fluorobenzyl)-1-(1-methyl-d3-piperidin-2,2,6,4-d4-4-yl)urea of formula X-D7

Using the procedure described in Example 3 with the use of the deuterated amine **II-D7** the product with the melting point of 112-116°C was obtained in the yield of 69%.

### Example 12

### Sodium hydroxy(4-isobutoxyphenyl)methanesulfonate of formula XI

Crude 4-isobutoxybenzaldehyde (**VI**; 57 g, 0.32 mol) was dissolved in ethanol (600 ml) and under stirring a solution of Na₂S₂O₅ (30 g, 0.16 mol) in water (60 ml) was added. The solid fraction immediately started to get separated, the mixture was stirred for 6 hours at 35°C under nitrogen. The separated fraction was aspirated, washed with ethanol and dried in air at the laboratory temperature. The amount of 81.2 g (90%) of a white product with the melting point of 151-155°C was obtained. ¹H NMR (DMSO-*d6*, 500.13 MHz, 298 K): 7.33 (d, 2H, *J* = 8.6, Ar*H*), 6.79 (d, 2H, *J* = 8.6, Ar*H*), 5.77 (d, 1H, *J* = 5.1, O*H*), 4.90 (d, 1H, *J* = 5.1, C*H*-OH), 3.71 (d, 2H, *J* = 6.7, C*H₂*), 1.99 (m, 1H, (CH₃)₂-C*H*), 0.97 (d, 6H, *J* = 6.7, (C*H₃*)₂). ¹³C NMR (CDCl₃, 125.76 MHz, 298 K): 157.85 (quart Ar*C*), 131.58 (quart Ar*C*), 128.93 (Ar*C*H), 112.97 (Ar*C*H), 84.56 (*C*H-OH), 73.70 (*C*H₂), 52.32 N-*C*H), 27.72 ((CH₃)₂-*C*H), 19.11 ((*C*H₃)₂).

### Example 13

### 4-isobutoxybenzaldehyde dimethyl acetal of formula XII

Crude 4-isobutoxybenzaldehyde (**VI**; 17.8 g, 0.1 mol) was dissolved in methanol (200 ml) and trimethyl orthoformate (100 ml) and after addition of the saturated solution of HCl in methanol (10 ml) the mixture was stirred at the laboratory temperature for 48 hours. After addition of triethylamine (10 ml) the mixture was concentrated in vacuum, diluted with ethyl acetate (200 ml) and washed with water (2 x 50 ml) and salt brine (50 ml) and dried with magnesium sulphate. The amount of 19.6 g (87%) of the brownish evaporation product was obtained, which was used for the next reaction stage without further purification. ¹H NMR spectrum (CDCl₃): ¹H NMR (CDCl₃, 500.13 MHz, 298 K): 7.83 (d, 2H, *J* = 8.8 Hz, Ar*H*), 6.99 (d, 2H, *J* = 8.8 Hz, Ar*H*), 5.44 (s, 1H, C*H*), 3.80 (d, 2H, *J* = 6.6 Hz, -C*H₂*-CH-(CH₃)₂), 3.26 (s, 6H, C*H₃*O), 2.12 (nonet, 1H, *J* = 6.7 Hz, -CH₂-C*H*-(CH₃)₂), 1.04 (d, 6H, *J* = 6.7 Hz, -CH₂-CH-(C*H₃*)₂).

### Example 14

### 1-(4-Fluorobenzyl)-3-(4-isobutoxybenzyl)-1-(1-methylpiperidin-4-yl)urea of formula I

To a solution of 4-isobutoxybenzaldehyde (**VI**; 0.18 g, 1 mmol) in THF (2 ml) 1-(4-fluorobenzyl)-3-(4-isobutoxybenzyl)-1-(1-methylpiperidin-4-yl)urea (**X**; 0.32 g, 1.2 mmol) was added and the resulting suspension was stirred in a vial under nitrogen for 30 minutes. Then, Ti(O-iPr)₄ (0.5 g, 1.8 mmol) was added and the mixture was stirred at the laboratory temperature for 24 h. Then, NaBH₄ (0.1 g, 2.6 mmol) was added and the mixture was stirred for another 2 h at the laboratory temperature. After cooling to 0°C 1M-HCl (4 ml) was added dropwise and then water (4 ml). The mixture was washed with ethyl acetate, the aqueous layer was alkalinized to pH 10 and extracted with dichloromethane (5 x 2 ml). The organic layer was washed with salt brine (2 ml) and dried with MgSO₄. The obtained evaporation product (0.17 g) contained 3% of 4-isobutoxybenzaldehyde (**VI**), 4% of 1-(4-fluorobenzyl)-3-(4-isobutoxy-benzyl)-1-(1-methylpiperidin-4-yl)urea (**X**) and 51% of pimavanserin according to HPLC. A division by means of preparative TLC provided 55 mg of pimavanserin (HPLC, NMR, MS).

### Example 15

### 1-(4-Fluorobenzyl)-3-(4-isobutoxybenzyl)-1-(1-methylpiperidin-4-yl)urea of formula I

To a solution of 4-isobutoxybenzaldehyde (**VI**; 0.18 g, 1 mmol) in THF (2,5 ml) 1-(4-fluorobenzyl)-3-(4-isobutoxybenzyl)-1-(1-methylpiperidin-4-yl)urea (**X**; 0.27 g, 1 mmol) was added and the resulting suspension was stirred under nitrogen for 30 minutes. Then, Ti(O-iPr)₄ (0.5 g, 1.8 mmol) was added and the mixture was stirred at the laboratory temperature for 24 h. Then, NaBH₄ (0.1 g, 2.6 mmol) was added and the mixture was stirred for another 12 h at the laboratory temperature. After cooling to 0°C, water (5 ml) was added and then 1M-HCl dropwise to achieve pH 2-3. The mixture was washed with ethyl acetate, the aqueous layer was alkalinized to pH 10 and extracted with dichloromethane (5 x 2 ml). The organic layer was washed with salt brine (2 ml) and dried with MgSO₄. The obtained evaporation product (0.25 g) still contained 6% of 4-isobutoxybenzaldehyde (**VI**), 6% of 1-(4-fluorobenzyl)-3-(4-isobutoxy-benzyl)-1-(1-methylpiperidin-4-yl)urea (**X**) and 44% of pimavanserin according to HPLC. A division by means of preparative TLC provided 70 mg of pimavanserin (HPLC, NMR, MS).

### Example 16

### 1-(4-Fluorobenzyl)-3-(4-isobutoxybenzyl)-1-(1-methylpiperidin-4-yl)urea of formula I

To a solution of 4-isobutoxybenzaldehyde (**VI**; 0.18g, 1 mmol) in acetic acid (2 ml) 1-(4-fluorobenzyl)-3-(4-isobutoxybenzyl)-1-(1-methylpiperidin-4-yl)urea (**X**; 0.27, 1 mmol) was added and the resulting suspension was stirred in a vial under nitrogen for 30 minutes until dissolution. Then, TMSCl (0.33 g, 3 mmol) was added dropwise and the mixture was stirred at the laboratory temperature for 24 h. Then, NaBH₄ (0.1 g, 2.6 mmol) was added and the mixture was stirred for another 2 h at the laboratory temperature. After addition of water (2 ml) the mixture was stirred for 2 h, evaporated until dry and the evaporation product was alkalinized with NaOH to pH 10. The solution obtained by extraction with dichloromethane (5 x 2 ml) was washed with salt brine (2 ml) and dried with MgSO₄. The obtained evaporation product (0.28 g) still contained 3% of 4-isobutoxybenzaldehyde (**VI**), 22% of 1-(4-fluorobenzyl)-3-(4-isobutoxy--benzyl)-1-(1-methylpiperidin-4-yl)urea (**X**) and 48% of pimavanserin according to HPLC. A division by means of preparative TLC provided 80 mg of pimavanserin (HPLC, NMR, MS).

### Example 17

### 1-(4-Fluorobenzyl)-3-(4-isobutoxybenzyl)-1-(1-methylpiperidin-4-yl)urea of formula I

To a solution of 4-isobutoxybenzaldehyde (**VI**; 0.18g, 1 mmol) in toluene (2 ml) 1-(4--fluorobenzyl)-3-(4-isobutoxybenzyl)-1-(1-methylpiperidin-4-yl)urea (**X**; 0.27, 1 mmol) was added and trifluoroacetic acid (0.3 g, 2.6 mmol) was added to the resulting suspension in a vial under nitrogen. Et₃SiH (0.35 g, 3 mmol) was then added to the obtained solution and the mixture was stirred at the laboratory temperature for 24 h. After dilution with toluene (5 ml) the solution was washed with the saturated aqueous solution of NaHCO₃ and then dried with MgSO₄. The obtained evaporation product (0.22 g) contained less than 1% of 4-isobutoxybenzaldehyde (**VI**) as well as 1-(4-fluorobenzyl)-3-(4-isobutoxy-benzyl)-1-(1-methyl-piperidin-4-yl)urea (**X**) and 78% of pimavanserin. A division by means of preparative TLC provided 130 mg of pimavanserin (HPLC, NMR, MS).

### Example 18

### 1-(4-Fluorobenzyl)-3-(4-isobutoxybenzyl)-1-(1-methylpiperidin-4-yl)urea of formula I

0.2 g of the evaporation product containing 77% of pimavanserin was obtained by means of the procedure described in Example 16 with the use of dichloromethane as the solvent.

### Example 19

### 1-(4-Fluorobenzyl)-3-(4-isobutoxybenzyl)-1-(1-methylpiperidin-4-yl)urea of formula I

To a solution of 4-isobutoxybenzaldehyde (**VI**; 0.18g, 1 mmol) in toluene (2 ml) 1-(4-fluorobenzyl)-3-(4-isobutoxybenzyl)-1-(1-methylpiperidin-4-yl)urea (**X**; 0.27, 1 mmol) was added and trifluoroacetic acid (0.3 g, 2.6 mmol) was added to the resulting suspension in a vial under nitrogen. Tetramethyldisiloxane (TMDS, 0.4 g, 3 mmol) was then added to the obtained solution and the mixture was stirred at the laboratory temperature for 24 h. After dilution with toluene (5 ml) the solution was washed with the saturated aqueous solution of NaHCO₃ and then dried with MgSO₄. The obtained evaporation product (0.24 g) contained 82% of pimavanserin according to HPLC.

### Example 20

### 1-(4-Fluorobenzyl)-3-(4-isobutoxybenzyl)-1-(1-methylpiperidin-4-yl)urea of formula I

To a solution of 4-isobutoxybenzaldehyde (**VI**; 0.18g, 1 mmol) in toluene (2 ml) 1-(4-fluorobenzyl)-3-(4-isobutoxybenzyl)-1-(1-methylpiperidin-4-yl)urea (**X**; 0.27, 1 mmol) was added and trifluoroacetic acid (0.5 ml) was added to the resulting suspension in a vial under nitrogen. Poly(methylhydrosiloxane) (PMHS, 0.2 g, Alfa Aesar, mol. w. 1900) was then added to the obtained solution and the mixture was stirred at 50°C for 48 h. After dilution with toluene (5 ml) the solution was washed with the saturated aqueous solution of NaHCO₃ and then dried with MgSO₄. The obtained evaporation product (0.22 g) contained 48% of pimavanserin according to HPLC.

### Example 21

### 1-(4-Fluorobenzyl)-3-(4-isobutoxybenzyl)-1-(1-methylpiperidin-4-yl)urea of formula I

To a solution of 4-isobutoxybenzaldehyde (**VI**; 0.25g, 1.4 mmol) in acetonitrile (2.5 ml) 1-(4-fluorobenzyl)-3-(4-isobutoxybenzyl)-1-(1-methylpiperidin-4-yl)urea (**X**; 0.27, 1 mmol) was added and trifluoroacetic acid (0.5 ml) was added to the resulting suspension in a vial under nitrogen. Poly(methylhydrosiloxane) (PMHS, 0.2 g, Alfa Aesar, mol. w. 1900) was then added to the obtained solution and the mixture was stirred at 50°C for 48 h. After evaporation to the dry state the evaporation product was dissolved in dichloromethane (10 ml), the resulting solution was washed with the saturated solution of NaHCO₃ (2 x 2 ml) and dried with MgSO₄. The obtained evaporation product (0.25 g) contained 52% of pimavanserin according to HPLC.

### Example 22

### 1-(4-Fluorobenzyl)-3-(4-isobutoxybenzyl)-1-(1-methylpiperidin-4-yl)urea of formula I

To a suspension of sodium hydroxy(4-isobutoxyphenyl)methansesulfonate (**XI**, 0.4 g, 1.4 mmol) in acetonitrile (4 ml) 1-(4-fluorobenzyl)-3-(4-isobutoxybenzyl)-1-(1-methylpiperidin-4-yl)urea (**X**; 0.27, 1 mmol) was added and trifluoroacetic acid (1 ml) was added to the obtained suspension in a vial under nitrogen. Et₃SiH (0.35 g, 3 mmol) was then added to the obtained solution and the mixture was stirred at the temperature of 50°C for 24 h. After evaporation to the dry state the evaporation product was triturated with dichloromethane (2 x 10 ml), the resulting solution was washed with the saturated solution of NaHCO₃ (2 x 2 ml) and dried with MgSO₄. The obtained evaporation product (0.22 g) contained 74% of pimavanserin according to HPLC.

### Example 23

### 1-(4-Fluorobenzyl)-3-(4-isobutoxybenzyl)-1-(1-methylpiperidin-4-yl)urea of formula I

To a solution of 4-isobutoxybenzaldehyde dimethyl acetal (**XII**; 0.32 g, 1.4 mmol) in acetonitrile (2.5 ml) 1-(4-fluorobenzyl)-3-(4-isobutoxybenzyl)-1-(1-methylpiperidin-4-yl)urea (**X**; 0.27, 1 mmol) was added and trifluoroacetic acid (0.5 ml) was added to the resulting suspension in a vial under nitrogen and the mixture was stirred at the laboratory temperature for 24 hours. Et₃SiH (0.35 g, 3 mmol) was then added to the obtained solution and the mixture was stirred at the temperature of 50°C for 8 h. After evaporation to the dry state the evaporation product was triturated with dichloromethane (2 x 10 ml), the resulting solution was washed with the saturated solution of NaHCO₃ (2 x 2 ml) and dried with MgSO₄. The obtained evaporation product (0.28 g) contained 62% of pimavanserin according to HPLC.

### Example 24

### 1-(4-Fluorobenzyl)-3-(4-isobutoxybenzyl)-1-(1-methylpiperidin-4-yl)urea of formula I

To a solution of 4-isobutoxybenzaldehyde (**VI**; 0.25g, 1.4 mmol) in dichloromethane (2.5 ml) 1-(4-fluorobenzyl)-3-(4-isobutoxybenzyl)-1-(1-methylpiperidin-4-yl)urea (**X**; 0.27, 1 mmol) was added and trifluoroacetic acid (0.5 ml) was added to the resulting suspension in a vial under nitrogen. Tetramethyldisiloxane (TMDS, 0.4 g, 3 mmol) was then added to the obtained solution and the mixture was stirred at the laboratory temperature for 24 h. After dilution with dichloromethane (5 ml) the solution was washed with the saturated aqueous solution of NaHCO₃ and then dried with MgSO₄. The obtained evaporation product (0.26 g) contained 77% of pimavanserin according to HPLC.

### Example 25

### 1-(4-Fluorobenzyl)-3-(4-isobutoxybenzyl)-1-(1-methylpiperidin-4-yl)urea of formula I

To a suspension of *N*-(4-fluorobenzyl)-1-methylpiperidine-4-amine dihydrochloride (**II.2HCl;** 7.4 g, 25 mmol) in methanol (50 ml) KCNO (4 g, 50 mmol) was added and the mixture was stirred for 4 h at the laboratory temperature. The insoluble fraction was aspirated, washed with methanol (10 ml) and the filtrate was concentrated to a quarter of the volume. Then, acetonitrile (25 ml) was added and 15 ml of mixture was removed by distillation. After addition of acetonitrile (15 ml) the amount of 15 ml of the mixture was removed by distillation. To the obtained concentrated product acetonitrile (50 ml) was added and the turbid solution was filtered. 4-isobutoxybenzaldehyde (**VI**; 6.25, 35 mmol) and trifluoroacetic acid (15 ml) were added to the clear solution. Tetramethyldisiloxane (TMDS, 5.4 g, 40 mmol) was then added to the obtained solution and the mixture was refluxed for 4 h while during the last hour of the reflux a part of the solvent (35 ml) was removed from the reaction mixture by distillation. Then, methanol (20 ml) was added to the reaction mixture, the mixture was refluxed for 1 h and then the reaction mixture was evaporated in vacuum. The saturated solution of NaHCO₃ was added to the mixture so that the mixture could achieve pH 10 (20 ml) and then it was stirred for 30 minutes (CO₂ being released). After addition of ethyl acetate (25 ml) the mixture was vigorously stirred for 1 h and after division the aqueous layer was extracted with ethyl acetate (5 x 25 ml). The united extracts were dried with MgSO₄ and the obtained evaporation product (10.5 g) contained 88% of pimavanserin according to HPLC. Finely spread D,L-tartaric acid (3.75 g) was added to a solution of crude pimavanserin in ethanol (50 ml) and the mixture was stirred at the laboratory temperature for 48 hours. The amount of 10.3 g (61%) of the D,L-tartrate (1:1) with the melt. point of 148-151°C and HPLC purity of 98.3% was obtained. ¹H NMR (DMSO-*d6*, 500 MHz, 298 K): 7.24 (dd, 1H, *J* = 8.6, 5.6 Hz, F-ArH), 7.12 (t, 1H, *J* = 8.8 Hz, F-ArH), 7.10 (d, 2H, *J* = 8.7 Hz, ArH), 6.93 (t, 1H, *J* = 5.8 Hz, NH), 6.83 (d, 2H, *J* = 8.7 Hz, ArH), 4.41 (s, 2H, N-C*H₂*-Ar-F), 4.18 (d, 2H, *J* = 5.7 Hz, C*H₂*-NH), 4.04 (s, 2H, tart-C*H*), 4.02 - 3.99 (m, 1H, N-C*H*-(CH₂)₂), 3.70 (d, 2H, *J* = 6.6 Hz, (CH₃)₂-CH-C*H₂*), 3.03 - 2.96 (m, 2H, CH₃-N-(C*H₂*)₂), 2.42 - 2.33 (m, 2H, CH₃-N-(C*H₂*)₂), 2.37 (s, 3H, C*H₃*-N), 1.99 (nonet, 1H, *J* = 6.6 Hz, (CH₃)₂-C*H*-CH₂), 1.75 - 1.63 (m, 2H, N-C*H*-(CH₂)₂), 1.55 - 1.49 (m, 2H, N-CH-(C*H₂*)₂), 0.97 (d, 6H, *J* = 6.7 Hz, (C*H₃*)₂-CH-CH₂). ¹³C NMR (DMSO-*d6*, 500 MHz, 298 K): 173.87 (tart-*C*=O), 160.92 (C-F), 157.44 (N-CO-NH and (CH₃)₂-CH-CH₂-O-*C*), 136.72 (quart. F-Ar*C*), 132.95 (quart. Ar*C*), 128.33 (F-Ar*C*H), 128.17 (Ar*C*H), 114.85 (F-Ar*C*H), 114.05 (Ar*C*H), 73.71 ((CH₃)₂-CH-*C*H₂-O), 71.64 (tart-*C*H), 53.96 (CH₃-N-(*C*H₂)₂), 51.14 (N-*C*H-(CH₂)₂), 44.14 (N-*C*H₂-Ar-F), 44.05 (N-*C*H₃), 43.08 (*C*H₂-NH), 28.48 (N-CH-(*C*H₂)₂), 27.71 ((CH₃)₂-*C*H-*C*H₂-O), 19.09 ((*C*H₃)₂-CH-CH₂-O).

A sample of the salt was converted in a standard manner to the base whose sample obtained by crystallization from ethanol with the melt. point of 116-118°C had the following NMR characteristics:
¹H NMR (DMSO-*d6*, 500 MHz, 298 K): 7.24 (dd, 1H, *J* = 8.6, 5.7 Hz, F-Ar*H*), 7.11 (t, 1H, *J* = 8.8 Hz, F-Ar*H*), 7.10 (d, 2H, *J* = 8.4 Hz, Ar*H*), 6.88 (t, 1H, *J* = 5.9 Hz, N*H*), 6.83 (d, 2H, *J* = 8.6 Hz, Ar*H*), 4.41 (s, 2H, N-C*H₂*-Ar-F), 4.18 (d, 2H, *J* = 5.8 Hz, C*H₂*-NH), 3.98 - 3.89 (m, 1H, N-C*H*-(CH₂)₂), 3.70 (d, 2H, *J* = 6.6 Hz, (CH₃)₂-C*H*-CH₂), 2.82 - 2.73 (m, 2H, CH₃-N-(C*H₂*)₂), 2.16 (s, 3H, C*H₃*-N), 2.04 - 1.94 (m, 2H, CH₃-N-(C*H₂*)₂), 1.99 (nonet, 1H, *J* = 6.7 Hz, (CH₃)₂-C*H*-CH₂), 1.64 - 1.53 (m, 2H, N-CH-(C*H*₂)₂), 1.49 - 1.41 (m, 2H, N-CH-(C*H*₂)₂), 0.97 (d, 6H, J = 6.7 Hz, (C*H₃*)₂-CH-CH₂). ¹³C NMR (DMSO-*d6*, 500 MHz, 298 K): 160.83 (*C*-F), 157.47 (N-CO-NH or (CH₃)₂-CH-CH₂-O-*C*), 157.40 (N-*C*O-NH or (CH₃)₂-CH-CH₂-O-*C*), 136.96 (quart. F-Ar*C*), 133.02 (quart. Ar*C*), 128.8.0 (F-Ar*C*H), 128.11 (Ar*C*H), 114.72 (F-Ar*C*H), 114.02 (Ar*C*H), 73.71 ((CH₃)₂-CH-*C*H₂-O), 54.62 (CH₃-N-(*C*H₂)₂), 52.01 (N-*C*H-(CH₂)₂), 45.34 (N-*C*H₃), 43.95 (N-*C*H₂-Ar-F), 43.05 (*C*H₂-NH), 29.54 (N-CH-(*C*H₂)₂), 27.67 ((CH₃)₂-*C*H-*C*H₂-O), 19.03 ((*C*H₃)₂-CH-CH₂-O).

## Claims

1. A method for preparing the pimavanserin of formula (**I**), 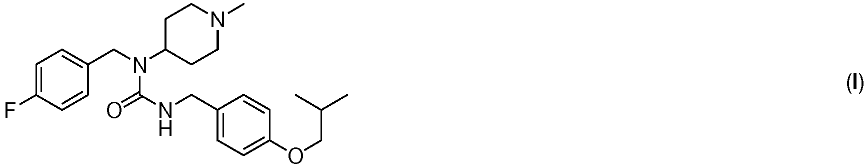 ***characterized in that*** it comprises the following steps:
a) a reaction of *N*-(4-fluorobenzyl)-1-methylpiperidine-4-amine of formula **II** or its salts 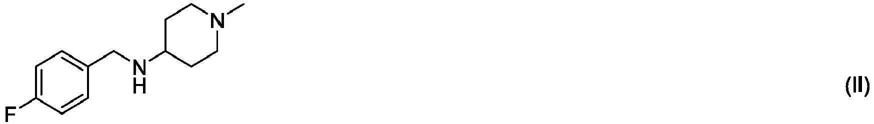 with isocyanic acid or its salt, producing 1-(4-fluorobenzyl)-1-(1-methylpiperidin-4-yl)urea of formula **X**, 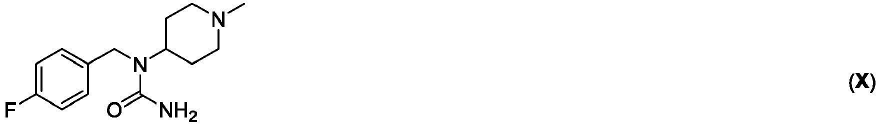
b) a reaction of 1-(4-fluorobenzyl)-1-(1-methylpiperidin-4-yl)urea of formula X with 4-isobutoxybenzaldehyde of formula **VI**, 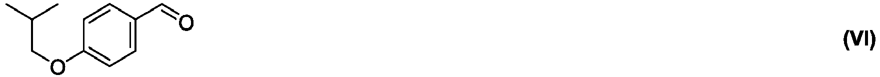 or its precursors such as the bisulphidic adduct of formula **XI** or acetals of formula **XII**, 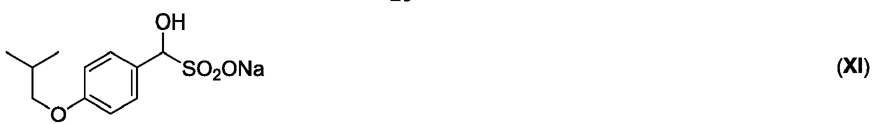 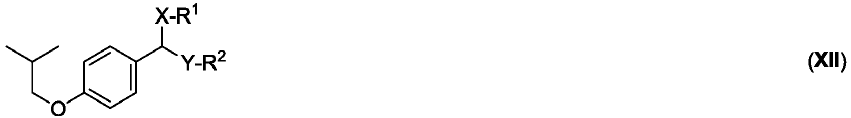
wherein X and Y can be independently O or S and wherein R¹ and
R² can be a C1-C6 alkyl, or wherein R¹, R² can form a 5- to 6-membered cycle together with the X-C-Y structure they are connected to.

2. The method for preparing in accordance with claim 1, ***characterized in that*** isocyanic acid from step a) is generated *in situ* from alkaline iso cyanates, preferably KNCO or NaNCO, with an acid selected from organic or inorganic acids.

3. The method for preparing in accordance with claim 1 or 2, ***characterized in that*** the reaction from step a) is carried out in organic solvents, in water or in a mixture of water with organic solvents.

4. The method for preparing in accordance with claim 3, ***characterized in that*** the organic solvent is selected from the group comprising aromatic hydrocarbons, C1-C5 alkyl alcohols, C1-C5 carboxylic acids, aprotic solvents such as dialkylamides of carboxylic acids, nitriles of carboxylic acids, or sulfoxides, preferably toluene, dimethyl formamide, dimethyl acetamide, *N*-methylpyrrolidone, acetonitrile, dimenthyl sulfoxide and the use of a mixture of one or more said solvents with water, wherein the content of water is in the whole concentration range, i.e. from 0 to 100%.

5. The method for preparing in accordance with claim 1 or 2, ***characterized in that*** the reaction from step a) is carried out with *N*-(4-fluorobenzyl)-1-methylpiperidine-4-amine dihydrochloride of formula **II** in an organic solvent, in a mixture of organic solvents, or in a mixture of an organic solvent and water, or in water.

6. The method for preparing in accordance with claim 5, ***characterized in that*** the reaction from step a) with *N*-(4-fluorobenzyl)-1-methylpiperidine-4-amine dihydrochloride of formula **II** is carried out in C1-C5 alkyl alcohols, preferably in methanol.

7. The method for preparing in accordance with claim 6, ***characterized in that*** the reaction from step a) is carried out at a temperature of 0 to 75°C, preferably at a temperature from 20 to 50°C, most preferably at the laboratory temperature.

8. The method for preparing in accordance with claim 1, ***characterized in that*** the compound of formula **X** in step b) is reacted with 4-isobutoxybenzaldehyde of formula **VI** in the presence of Ti compounds, preferably titanium alkoxides, most preferably with Ti(Oi-Pr)₄, with the use of a suitable reduction agent, preferably NaBH₄ or reduction agents derived from it as NaBH₃CN or NaBH(OAc)₃.

9. The method for preparing in accordance with claim 1, ***characterized in that*** the compound of formula **X** in step b) is reacted with 4-isobutoxybenzaldehyde of formula **VI** in the presence of Si compounds, preferably trialkyl silyl halides, most preferably with Me₃SiCl, with the use of a suitable reduction agent, preferably NaBH₄ or reduction agents derived from it as NaBH₃CN or NaBH(OAc)₃.

10. The method for preparing in accordance with claim 1, ***characterized in that*** the compound of formula **X** in step b) is reacted with 4-isobutoxybenzaldehyde of formula **VI** in the presence of reducing Si compounds, preferably reductive agents containing Si-H as the reductive group, more preferably trialkylsilanes such as triethylsilane, tetraalkyldisiloxanes, preferably tetramethyldisiloxane (TMDS), pentaalkyldisiloxanes, preferably pentamethyldisiloxane, cyclic hydrosiloxanes, preferably 2,4,6,8-tetramethylcyclotetrasiloxane or 2,4,6,8,10-pentamethylcyclopentasiloxane, or polymeric siloxanes containing the Si-H group, preferably polymethylhydrosiloxane (PMHS).

11. The method for preparing in accordance with claim 10, ***characterized in that*** the Si-H groups containing reducing agents activated by Lewis acids, preferably Ti(Oi-Pr)₄, BF₃, BCl₃, FeCl₃, InCl₃, BiCl₃, or Bronsted acids, preferably CF₃COOH or CF₃SO₂OH, are used.

12. The method for preparing in accordance with claims 1 to 11, ***characterized in that*** it further comprises the step of purification of pimavanserin of formula I by conversion to a salt with organic acids, preferably with pivalic, oxalic, succinic, maleic, fumaric, tartaric or citric acid, more preferably with tartaric acid, and the resulting salt is subsequently crystallized from solvents selected from the group comprising alcohols, esters of aliphatic acids, amides, ethers, or mixtures of these solvents, preferably from methanol, ethanol, 1-propanol, 2-propanol, 1-butanol, 2-methoxyethanol, methyl acetate, ethyl acetate, isopropyl acetate, *N*,*N*-dimethyl formamide, *N,N*-dimethyl acetamide, 1-methylpyrrolidone, tetrahydrofuran, dioxane, 1,2-dimethoxyethane, 1-methoxy-2-(2-methoxyethoxy)ethane or their mixture.

## Patentansprüche

1. Verfahren zur Herstellung von Pimavanserin von Formel (I) 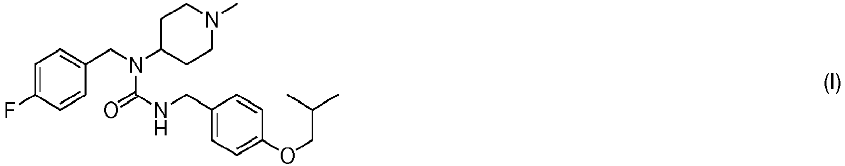 **dadurch gekennzeichnet, dass** es die folgenden Schritte beinhaltet:
a) eine Reaktion von *N*-(4-Fluorbenzyl)-1-methylpiperidin-4-amin von Formel II oder seinen Salzen 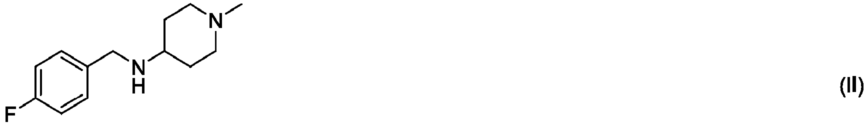 mit Isocyansäure oder ihrem Salz, so dass 1-(4-Fluorbenzyl)-1-(1-methylpiperidin-4-yl)urea von Formel X produziert wird, 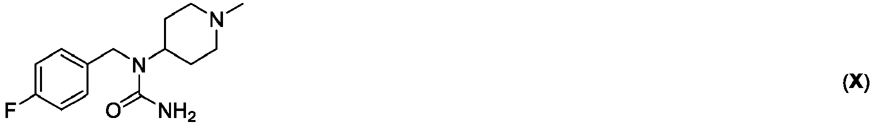
b) eine Reaktion von 1-(4-Fluorbenzyl)-1-(1-methylpiperidin-4-yl)urea von Formel X mit 4-Isobutoxybenzaldehyd von Formel VI 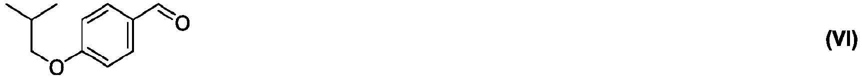 oder seinen Vorläufern, wie das Bisulfidaddukt von Formel XI oder Acetale von Formel XII 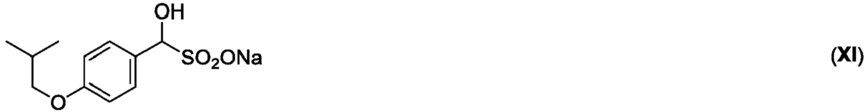 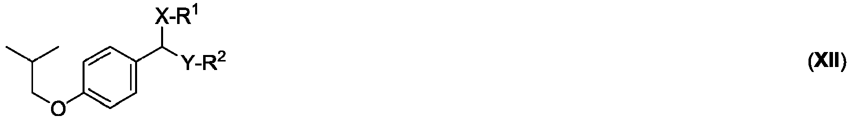 wobei X und Y unabhängig O oder S sein können und wobei R¹ und R² ein C1-C6 Alkyl sein können, oder wobei R¹, R² zusammen mit der X-C-Y Struktur, mit der sie verbunden sind, einen 5- bis 6-gliedrigen Cyclus bilden können.

2. Herstellungsverfahren nach Anspruch 1, **dadurch gekennzeichnet, dass** Isocyansäure aus Schritt a) *in situ* aus alkalischen Isocyanaten, vorzugsweise KNCO oder NaNCO, mit einer Säure, ausgewählt aus organischen oder anorganischen Säuren, erzeugt wird.

3. Herstellungsverfahren nach Anspruch 1 oder 2, **dadurch gekennzeichnet, dass** die Reaktion aus Schritt a) in organischen Lösungsmitteln, in Wasser oder in einem Gemisch aus Wasser und organischen Lösungsmitteln durchgeführt wird.

4. Herstellungsverfahren nach Anspruch 3, **dadurch gekennzeichnet, dass** das organische Lösungsmittel ausgewählt wird aus der Gruppe bestehend aus aromatischen Kohlenwasserstoffen, C1-C5 Alkylalkoholen, C1-C5 Carbonsäuren, aprotischen Lösungsmitteln, wie Dialkylamide von Carbonsäuren, Nitrile von Carbonsäuren oder Sulfoxide, vorzugsweise Toluol, Dimethylformamid, Dimethylacetamid, N-Methylpyrrolidon, Acetonitril, Dimethylsulfoxid, und ein Gemisch aus einem oder mehreren der genannten Lösungsmittel mit Wasser verwendet wird, wobei der Gehalt an Wasser im gesamten Konzentrationsbereich, d.h. von 0 bis 100 %, liegt.

5. Herstellungsverfahren nach Anspruch 1 oder 2, **dadurch gekennzeichnet, dass** die Reaktion aus Schritt a) mit *N*-(4-Fluorbenzyl)-1-methylpiperidin-4-amindihydrochlorid von Formel II in einem organischen Lösungsmittel, in einem Gemisch organischer Lösungsmittel oder in einem Gemisch aus einem organischen Lösungsmittel und Wasser oder in Wasser durchgeführt wird.

6. Herstellungsverfahren nach Anspruch 5, **dadurch gekennzeichnet, dass** die Reaktion aus Schritt a) mit N-(4-Fluorbenzyl)-1-methylpiperidin-4-amin-dihydrochlorid von Formel II in C1-C5 Alkylalkoholen, vorzugsweise in Methanol, durchgeführt wird.

7. Herstellungsverfahren nach Anspruch 6, **dadurch gekennzeichnet, dass** die Reaktion aus Schritt a) bei einer Temperatur von 0 bis 75°C, vorzugsweise bei einer Temperatur von 20 bis 50°C, am bevorzugtesten bei der Labortemperatur, durchgeführt wird.

8. Herstellungsverfahren nach Anspruch 1, **dadurch gekennzeichnet, dass** die Verbindung von Formel X in Schritt b) mit 4-Isobutoxybenzaldehyd von Formel VI in Anwesenheit von Ti-Verbindungen, vorzugsweise Titanalkoxide, am bevorzugtesten mit Ti(Oi-Pr)₄, unter Verwendung eines geeigneten Reduktionsmittels, vorzugsweise NaBH₄ oder davon abgeleitete Reduktionsmittel wie NaBH₃CN oder NaBH(OAc)₃, zur Reaktion gebracht wird.

9. Herstellungsverfahren nach Anspruch 1, **dadurch gekennzeichnet, dass** die Verbindung von Formel X in Schritt b) mit 4-Isobutoxybenzaldehyd von Formel VI in Anwesenheit von Si-Verbindungen, vorzugsweise Trialkylsilylhalogenide, am bevorzugtesten mit Me₃SiCl, unter Verwendung eines geeigneten Reduktionsmittels, vorzugsweise NaBH₄ oder davon abgeleitete Reduktionsmittel wie NaBH₃CN oder NaBH(OAc)₃, zur Reaktion gebracht wird.

10. Herstellungsverfahren nach Anspruch 1, **dadurch gekennzeichnet, dass** die Verbindung von Formel X in Schritt b) mit 4-Isobutoxybenzaldehyd von Formel VI in Anwesenheit reduzierender Si-Verbindungen, vorzugsweise Reduktionsmittel, die Si-H als reduzierende Gruppe enthalten, bevorzugter Trialkylsilane wie Triethylsilan, Tetraalkyldisiloxane, vorzugsweise Tetramethyldisiloxan (TMDS), Pentaalkyldisiloxane, vorzugsweise Pentamethyldisiloxan, cyclische Hydrosiloxane, vorzugsweise 2,4,6,8-Tetramethylcyclotetrasiloxan oder 2,4,6,8,10-Pentamethylcyclopentasiloxan oder polymere Siloxane, die die Si-H Gruppe enthalten, vorzugsweise Polymethylhydrosiloxan (PMHS), zur Reaktion gebracht wird.

11. Herstellungsverfahren nach Anspruch 10, **dadurch gekennzeichnet, dass** die Si-H Gruppen enthaltenden Reduktionsmittel, aktiviert durch Lewis-Säuren, vorzugsweise Ti(Oi-Pr)₄, BF₃, BCl₃, FeCl₃, InCl₃, BiCl₃, oder Brönsted-Säuren, vorzugsweise CF₃COOH oder CF₃SO₂OH, verwendet werden.

12. Herstellungsverfahren nach den Ansprüchen 1 bis 11, **dadurch gekennzeichnet, dass** es ferner den Schritt der Reinigung von Pimavanserin von Formel I durch Umwandeln in ein Salz mit organischen Säuren, vorzugsweise mit Pivalin-, Oxal-, Bernstein-, Malein-, Fumar-, Wein- oder Zitronensäure, bevorzugter mit Weinsäure, beinhaltet und das resultierende Salz anschließend aus Lösungsmitteln kristallisiert wird, die ausgewählt werden aus der Gruppe bestehend aus Alkoholen, Estern von aliphatischen Säuren, Amiden, Ethern oder Gemischen dieser Lösungsmittel, vorzugsweise aus Methanol, Ethanol, 1-Propanol, 2-Propanol, 1-Butanol, 2-Methoxyethanol, Methylacetat, Ethylacetat, Isopropylacetat, *N*,*N*-Dimethylformamid, N,N-Dimethylacetamid, 1-Methylpyrrolidon, Tetrahydrofuran, Dioxan, 1,2-Dimethoxyethan, 1-Methoxy-2-(2-methoxyethoxy)ethan oder ihrem Gemisch.

## Revendications

1. Procédé de préparation de la pimavansérine de formule **(I),** 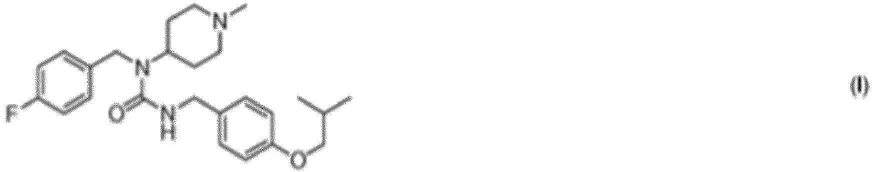 *caractérisé en ce qu*'il comprend les étapes suivantes :
a) une réaction entre de la N-(4-fluorobenzyl)-1-méthylpipéridin-4-amine de formule **II** ou ses sels 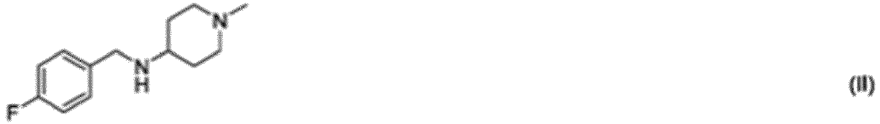 avec de l'acide isocyanique ou son sel, produisant 1-(4-fluorobenzyl)-1-(1-méthylpipéridin-4-yl)urée de formule **X**, 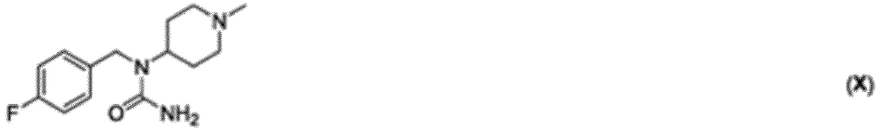
b) une réaction entre la 1-(4-fluorobenzyl)-1-(1-méthylpipéridin-4-yl)urée de formule X avec du 4-isobutoxybenzaldéhyde de formule **VI,** 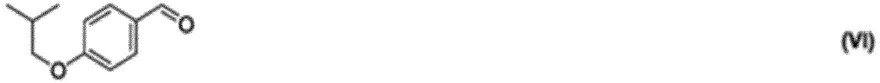 ou ses précurseurs tel que l'adduit de bisulfite de formule XI ou acétals de formule **XII**, 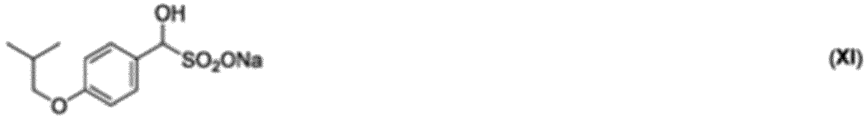 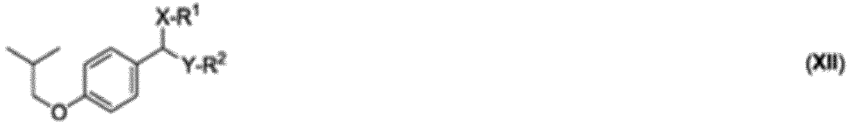 dans lequel X et Y peuvent être indépendamment O ou S et dans lequel R¹ et R² peuvent être un alkyle Cl-C6, ou dans lequel R¹, R² peuvent former un cycle à 5 à 6 éléments avec la structure X-C-Y à laquelle ils sont connectés.

2. Procédé de préparation selon la revendication 1, ***caractérisé en ce que*** l'acide isocyanique de l'étape a) est généré *in situ* à partir d'isocyanates alcalins, de préférence KNCO ou NaNCO, avec un acide sélectionné parmi les acides organiques ou inorganiques.

3. Procédé de préparation selon la revendication 1 ou 2, ***caractérisé en ce que*** la réaction de l'étape a) est réalisée dans des solvants organiques, dans de l'eau ou dans un mélange d'eau et de solvants organiques.

4. Procédé de préparation selon la revendication 3, ***caractérisé en ce que*** le solvant organique est sélectionné dans le groupe comprenant des hydrocarbures aromatiques, des alcools alkyles Cl-C5, des acides carboxyliques Cl-C5, des solvants aprotiques tels que des dialkylamides d'acides carboxyliques, nitriles d'acides carboxyliques, ou sulfoxydes, de préférence toluène, diméthylformamide, diméthylacétamide, N-méthylpyrrolidone, acétonitrile, diméthylsulfoxide et l'utilisation d'un mélange desdits un ou plusieurs solvants avec de l'eau, dans lequel la teneur en eau est comprise dans la plage de concentration totale, c.-à-d. de 0 à 100 %.

5. Procédé de préparation selon la revendication 1 ou 2, ***caractérisé en ce que*** la réaction de l'étape a) est réalisée avec du dihydrochlorate de N-(4-fluorobenzyl)-1-méthylpiperidin-4-amine de formule **II** dans un solvant organique, dans un mélange de solvants organiques, dans un mélange d'un solvant organique et d'eau, ou dans l'eau.

6. Procédé de préparation selon la revendication 5, ***caractérisé en ce que*** la réaction de l'étape a) avec du dihydrochlorate de N-(4-fluorobenzyl)-1-méthylpipéridin-4-amine de formule **II** est réalisé dans des alcools alkyles Cl-C5, de préférence dans du méthanol .

7. Procédé de préparation selon la revendication 6, ***caractérisé en ce que*** la réaction de l'étape a) est réalisée à une température de 0 à 75 °C, de préférence à une température de 20 à 50 °C, idéalement à la température de laboratoire.

8. Procédé de préparation selon la revendication 1, ***caractérisé en ce* que** le composé de la formule **X** à l'étape b) est mis en réaction avec du 4-isobutoxybenzaldéhyde de formule **VI** en présence de composés Ti, de préférence des alkoxydes de titane, idéalement avec Ti(Oi-Pr)_{4,} en utilisant un agent de réduction convenable, de préférence NaBH₄ ou des agents de réduction dérivés de celui-ci comme NaBH₃CN ou NaBH(OAc)₃.

9. Procédé de préparation selon la revendication 1, ***caractérisé en ce que*** le composé de la formule **X** à l'étape b) est mis en réaction avec du 4-isobutoxybenzaldé de formule **VI** en présence de composés Si, de préférence des halogénures trialkylsilyles, idéalement avec Me₃SiCl, en utilisant un agent de réduction convenable, de préférence NaBH₄ ou des agents de réduction dérivés de celui-ci comme NaBH₃CN ou NaBH(OAc)₃.

10. Procédé de préparation selon la revendication 1, ***caractérisé en ce que*** le composé de la formule X à l'étape b) est mis en réaction avec du 4-isobutoxybenzaldééyde de formule **VI** en présence de composés Si de réduction, de préférence des agents réducteurs contenant Si-H comme groupe réducteur, idéalement des trialkylsilanes tels que triethylsilane, tétraalkyldisiloxanes, de préférence tétramethyldisiloxane **(TMDS),** pentaalkyldisiloxanes, de préférence pentaméthyldisiloxane, hydrosiloxanes cycliques, de préférence 2,4,6,8-tétraméthylcyclotétrasiloxane ou 2,4,6,8,10-pentaméthylcyclopentasiloxane, ou des siloxanes polymériques contenant le groupe Si-H, de préférence polyméthylhydrosiloxane **(PMHS).**

11. Procédé de préparation selon la revendication 10, ***caractérisé en ce que*** les groupes Si-H contenant des agents de réduction activés par des acides de Lewis, de préférence Ti(Oi-Pr)₄, BF₃, BCl₃, FeCl₃, InCl₃, BiCl₃, ou des acides de Brönsted, de préférence CF₃COOH ou CF₃SO₂OH sont utilisés.

12. Procédé de préparation selon la revendications 1 à 11, ***caractérisé en ce qu'***il comprend en outre l'étape de purification de la pimavansérine de la formule **I** par conversion en un sel avec des acides organiques, de préférence avec de l'acide pivalique, oxalique, succinique, maléique, fumarique, tartrique ou citrique, idéalement avec de l'acide tartrique, et le sel résultant est ensuite cristallisé à partir de solvants sélectionnés dans le groupe comprenant alcools, esters d'acides aliphatiques, amides, éthers, ou mélanges de ces solvants, de préférence parmi méthanol, éthanol, 1-propanol, 2-propanol, 1-butanol, 2-méthoxyéthanol, acétate de méthyle, acétate d'éthyle, acétate d'isopropyle, N,N-diméthylformamide, N,N-diméthylacetamide, 1-méthylpyrrolidone, tétrahydrofuranne, dioxane, 1,2-diméthoxyéthane, 1- méthoxy-2-(2-méthoxyéthoxy)éthane ou leur mélange.
